# EUROPÄISCHE PATENTANMELDUNG

(11) **EP 3 156 085 A1**
(43) Veröffentlichungstag der Anmeldung: **19.04.2017**
(21) Anmeldenummer: 15189439.1
(22) Anmeldetag: 12.10.2015
(51) Int. Cl.: A61M 1/10

(54) **HERZUNTERSTÜTZUNGSSYSTEM MIT ZWEI PUMPEN**

(71) Anmelder: Berlin Heart GmbH, 12247 Berlin (DE)
(72) Erfinder: NÜSSER, Peter, 14532 Kleinmachnow (DE); ARSLAN, Nedim, 10717 Berlin (DE); GRAICHEN, Kurt, 13189 Berlin (DE); MÜLLER, Jörg, 10439 Berlin (DE)
(74) Vertreter: Pfenning, Meinig & Partner mbB

(57) **Zusammenfassung**

Es wird ein Herzunterstützungssystem mit zwei Pumpen (1, 4) und mit diesen verbundenen Kanülen vorgestellt, wobei eine erste Pumpe zur Verbindung eines mittels zweier Kanülen (2, 3, 5, 6, 15, 16, 21, 22, 23, 26) gebildeten ersten Fluidkanals mit dem Rechtsherzsystem, insbesondere mit dem rechten Ventrikel und der Pulmonalarterie, eingerichtet ist, während die zweite Pumpe zur Verbindung eines mittels zweier weiterer Kanülen gebildeten zweiten Fluidkanals mit dem Linksherzsystem, insbesondere mit dem linken Ventrikel einerseits und der Aorta andererseits, eingerichtet ist.

Um eine gute Steuerbarkeit der Pumpen mit einem geringen Platzbedarf und Energieverbrauch und einer Einsetzbarkeit bei geringen Fluidkanaldurchmessern zu verbinden, ist die Verwendung von Rotationspumpen vorgesehen.

## Beschreibung

Die Erfindung liegt auf dem Gebiet der Mechanik und der Strömungsmechanik und ist mit besonderem Vorteil beispielsweise in der Medizintechnik einsetzbar.

Es sind seit einiger Zeit Herzunterstützungssysteme für Patienten bekannt, bei denen durch eine oder mehrere Herzunterstützungspumpen das Auswurfvolumen in einem oder beiden Kreisläufen eines Patientenkörpers unterstützt wird. Damit kann die Funktion des Patientenherzens teilweise und zeitweise oder andauernd unterstützt oder ersetzt werden. Insbesondere sind auf diesem Gebiet Unterstützungssysteme bekannt geworden, bei denen zwei Pumpen außerhalb des Patientenkörpers angeordnet und mittels Kanülen mit verschiedenen Kammern des Herzens einerseits und Blutgefäßen andererseits verbunden sind. Auf diese Weise können sowohl das Linksherzsystem als auch das Rechtsherzsystem abgestimmt aufeinander unterstützt werden. Pumpen, die für diesen Zweck benutzt werden, sind bisher oft als Membranpumpen mit pneumatischem Antrieb bekannt geworden, die eine relativ hohe Antriebsenergie erfordern und einen pulsatilen Betrieb der Blutförderung vorsehen.

Der Betrieb solcher Herzunterstützungssysteme ist jedoch an eine unterbrechungsfreie Energieversorgung gebunden, die nur entweder stationär erfolgen kann oder die Mitnahme von Energiespeichern in Form von relativ schweren und voluminösen Batterien/Akkumulatoren erfordert. Oft erfordern solche Systeme auch Pneumatikeinheiten oder andere Antriebseinheiten zum Antrieb von Membranpumpen.

Es sind zudem Herzunterstützungssysteme mit vollimplantierten, sehr kleinen Pumpen bekannt geworden, die vorübergehend oder dauerhaft zur Herzunterstützung verwendet werden.

Der vorliegenden Erfindung liegt vor dem Hintergrund des Standes der Technik die Aufgabe zugrunde, ein Herzunterstützungssystem mit Pumpen zu schaffen, die platzsparend angeordnet werden, mit einem möglichst geringen Energieverbrauch arbeiten und die Blutförderung im Körper des Patienten auch durch relativ kleine Querschnitte von Fluidkanälen bewirken.

Zur Lösung der Aufgabe ergibt sich ein Herzunterstützungssystem mit zwei Pumpen und mit diesen verbundenen Kanülen, wobei eine erste Pumpe zur Verbindung mittels eines wenigstens teilweise durch zwei Kanülen gebildeten ersten Fluidkanals mit dem Rechtsherzsystem, insbesondere mit dem rechten Ventrikel und der Pulmonalarterie, eingerichtet ist, während die zweite Pumpe zur Verbindung mittels eines wenigstens durch zwei weitere Kanülen gebildeten zweiten Fluidkanals mit dem Linksherzsystem, insbesondere mit dem linken Ventrikel einerseits und der Aorta andererseits, eingerichtet ist, wobei beide Pumpen als Rotationspumpen zur Anordnung außerhalb eines Patientenkörpers ausgebildet sind.

Durch die Verwendung einer oder zweier Rotationspumpen zur Blutförderung ergibt sich bei einer guten Steuerbarkeit des Drucks und des synchronisierten Fördervolumens ein relativ geringer Energieverbrauch. Als Rotationspumpen können beispielsweise Pumpen mit einem axial fördernden Rotor oder auch Pumpen mit einem radial fördernden Rotor oder eine Kombination beider Förderarten eingesetzt werden.

Da für die Verwendung von Kanülen mit im Querschnitt kleinen Fluidkanälen innerhalb der Pumpen ein relativ hohes Druckgefälle erzeugt werden sollte, werden derartige Rotationspumpen dafür mit hohen Drehzahlen betrieben. Die Drehzahlen sollen andererseits jedoch nicht zu weit ansteigen, um Blutschädigungen bei der Blutförderung zu vermeiden.

Deshalb kann es vorgesehen sein, dass im Verlauf von wenigstens einem Fluidkanal, der mit einer der Pumpen verbunden ist, ein Konnektor vorgesehen ist, in dem sich der Querschnitt des Fluidkanals mit wachsender Entfernung von der Pumpe verringert. Hierdurch ergibt sich, dass im Bereich der Pumpen die Fluidkanäle einen relativ großen Querschnitt aufweisen, so dass das Blut in diesem Bereich ohne allzu großen Strömungswiderstand gefördert werden kann. Eine Verengung des Fluidkanalquerschnitts ergibt sich nur für einen Teil der Fluidkanäle, beispielsweise für den Teil der Fluidkanäle, der innerhalb des Patientenkörpers angeordnet ist. Somit können in diesem Bereich im Querschnitt kleine Kanülen verwendet werden, die auch einfach implantierbar sind. Dies kommt insbesondere der Verwendung eines Herzunterstützungssystems bei Kindern oder Kleinkindern zugute.

Es wird somit ein Konnektor benötigt, der zwischen einem größeren Querschnitt des Fluidkanals im Bereich einer oder beider Pumpen und einem kleineren Querschnitt des Fluidkanals in dem von den Pumpen entfernten Bereich, beispielsweise innerhalb des Patientenkörpers, eine Verbindung herstellt. Hierzu kann der Querschnitt des Fluidkanals innerhalb des Konnektors beispielsweise konisch ausgebildet sein.

Der Konnektor selbst kann beispielsweise aus einem steifen Material, insbesondere einem Kunststoff oder Metall, beispielsweise Titan, bestehen. Er kann jedoch auch aus einem elastomeren Material, wie beispielsweise einem Silikonelastomer, bestehen.

Ein derartiger Konnektor verbindet üblicherweise eine erste Kanüle mit größerem Innen- und Außendurchmesser mit einer zweiten Kanüle, deren Innen- und Außendurchmesser geringer sind als die der ersten Kanüle. Die erste Kanüle ist dabei der Herzpumpe näher als die zweite Kanüle.

Um den Konnektor mit einer mit einer Pumpe verbundenen Kanüle oder direkt mit einem Pumpenanschluss im Bereich des Pumpeneinlasses oder Pumpenauslasses fluiddicht zu verbinden, ist beispielsweise ein Verbindungselement vorgesehen, das aus einem Elastomer besteht. Ein solches Verbindungselement kann eine ringförmige Muffe sein, die an ihren Enden jeweils mit Schnappringen versehen ist, die auf eine Kanüle oder einen Pumpenanschluss einerseits und andererseits auf ein Ende des Konnektors aufschnappbar sind. Die Durchmesser des Konnektors, der Pumpenanschlüsse und der Kanülen sowie des Verbindungselements sind vorteilhaft derart aufeinander abgestimmt, dass das Verbindungselement elastisch auf die übrigen Anschlüsse aufklemmbar ist. Damit wird eine Fluiddichtigkeit im Verbindungsbereich erreicht.

Zudem kann der Konnektor eine Kante oder Nut aufweisen, über die ein Schnappring des Verbindungselements derart aufgeklemmt wird, dass ein radial nach innen ragender Steg des Verbindungselements in die Nut oder hinter dem Steg des Konnektors einrastet. Hierdurch wird die Sicherheit der Fluiddichtigkeit noch weiter verbessert. Eine ähnliche Steg/Nut-Verbindung kann auch im Verbindungsbereich zwischen dem Verbindungselement und einem Pumpenanschluss oder einer Kanüle vorgesehen sein.

Es kann zudem vorgesehen sein, dass wenigstens eine der Pumpen mit einem Graft verbunden ist. Bevorzugt ist die mit einem Auslass der Blutpumpe verbundene Kanüle an ihrem der Blutpumpe abgewandten Ende mit einem Graft verbunden. Mit einem derartigen Graft kann die jeweilige Kanüle direkt mit einem Blutgefäß durch Vernähen verbunden werden.

Es kann außerdem vorgesehen sein, dass die Kanülen jeweils einen Bereich aufweisen, der zum Durchtritt durch die Haut des Patienten eingerichtet ist. Bevorzugt sind alle Kanülen des Herzunterstützungssystems durch die Haut des Patienten führbar oder geführt. Eine Kanüle oder beide Kanülen, die jeweils eine Pumpe und das Patientenherz miteinander verbinden, können beispielsweise in ihrem Außenbereich abschnittsweise aufgeraut sein, um ein Einwachsen der Haut des Patienten im Bereich des Durchtritts der Kanüle durch die Haut zu fördern. Anstelle einer Aufrauung kann auch eine Beschichtung mit einem Material stehen, das das Einwachsen in die Haut fördert.

Die erzeugten Druckgefälle der beiden Pumpen und die Fördermengen können gezielt aufeinander abgestimmt werden. Hierzu ist es jedoch sinnvoll, beide Pumpen durch ein gemeinsames Steuergerät anzusteuern.

Weiterhin ist es sinnvoll, einen oder zwei oder auch mehr Drucksensoren vorzusehen, mit denen das Steuergerät verbunden ist. Dabei kann wenigstens einer der Drucksensoren jeweils in einem der Fluidkanäle oder in einem Herzventrikel angeordnet sein, so dass die jeweils mit einer der Pumpen verbundenen Fluidkanäle bezüglich des Drucks und des Fördervolumens einzeln überwacht und die ihnen zugeordneten Pumpen geeignet angesteuert werden können.

Wie bereits oben ausgeführt, kann auch vorgesehen sein, dass wenigstens ein Konnektor unmittelbar an einem Pumpeneinlass oder -auslass angeordnet ist.

In den bisherigen Ausführungen wurde stets davon ausgegangen, dass das Herzunterstützungssystem zwei Rotationspumpen aufweist. Ein weiteres Herzunterstützungssystem gemäß dieser Beschreibung ist ein Herzunterstützungssystem mit einer Pumpe und mit dieser verbundenen Kanülen, wobei die Pumpe zur Verbindung mittels eines wenigstens teilweise durch wenigstens eine Kanüle gebildeten ersten Fluidkanals mit dem Rechtsherzsystem, insbesondere mit dem rechten Ventrikel und der Pulmonalarterie, eingerichtet ist oder zur Verbindung mittels eines wenigstens teilweise durch wenigstens eine Kanüle gebildeten zweiten Fluidkanals mit dem Linksherzsystem, insbesondere mit dem linken Ventrikel einerseits und der Aorta andererseits, eingerichtet ist, dadurch gekennzeichnet, dass die Pumpe als Rotationspumpe zur Anordnung außerhalb eines Patientenkörpers ausgebildet ist.

Es wird außer einem Herzunterstützungssystem auch ein Computerprogrammprodukt mit einem Programm zum Betrieb des Steuergeräts derart, dass der Druck und/oder die Volumenströme in den beiden mit je einer der Pumpen verbundenen Fluidkanälen aufeinander abgestimmt sind, vorgestellt. Dabei kann in einer Ausführungsform vorgesehen sein, dass der innerhalb der Pumpen anliegende Druck bzw. der innerhalb der Kanülen anliegende Druck den physiologischen Notwendigkeiten des jeweiligen Herzsystems, beispielsweise dem Links- oder Rechtsherzsystem, angepasst wird. Umfasst das Herzunterstützungssystem zwei Blutpumpen, so fördern beide im gleichen kurzen Zeitraum denselben Volumenstrom.

Ein solches Programm kann ein Verfahren zum Betrieb eines Herzunterstützungssystems so steuern, dass die Leistungen der beiden Pumpen derart gesteuert werden, dass der Druck und/oder die Volumenströme in den beiden mit je einer der Pumpen verbundenen Fluidkanälen aufeinander abgestimmt werden.

Durch eine geeignete periodisch schwankende Ansteuerung der Pumpen kann auch eine pulsatile Fördercharakteristik der Pumpen realisiert werden, die der natürlichen Fördercharakteristik eines gesunden Herzens näher kommt als ein relativ konstanter wenig pulsatiler Fluidstrom durch die Blutgefäße.

Im Folgenden werden das geschilderte Herzunterstützungssystem sowie das Verfahren zu seinem Betrieb anhand von Ausführungsbeispielen mithilfe der beigefügten Figuren einer Zeichnung detailliert dargestellt und erläutert. Dabei zeigt
- Fig. 1: schematisch den Körper eines Patienten mit dem Herzen des Patienten und zwei Herzpumpen,
- Fig. 2: eine Herzpumpe in einer Außenansicht,
- Fig. 3: eine weitere Herzpumpe mit einem radial fördernden Rotor in einer dreidimensionalen Ansicht,
- Fig. 4: einen konischen Konnektor mit Verbindungselementen sowie
- Fig. 5: einen konischen Konnektor mit Verbindungselementen, die Schnappringe aufweisen.

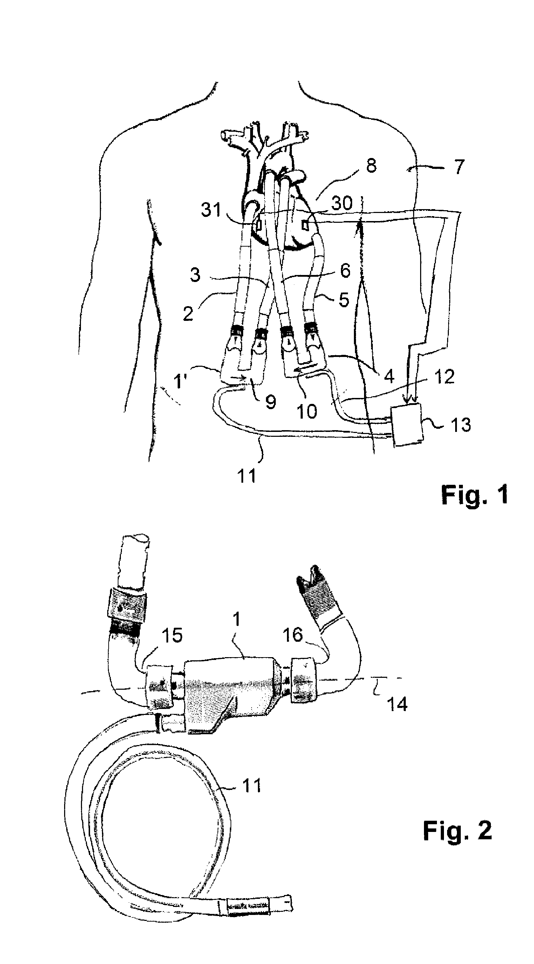

Figur 1 zeigt schematisch den Körper 7 eines Patienten mit seinem Herzen 8. An das Herz 8 sind mittels Kanülen 2, 3, 5, 6 zwei Herzpumpen 1, 4 angeschlossen. Dabei führt die Kanüle 2 vom rechten Atrium zur Pumpe 1, während die Kanüle 3 von der Pumpe 1 zur Pulmonalarterie führt. Die Kanüle 5 führt vom Apex zur zweiten Pumpe 4, und die Kanüle 6 führt von der Pumpe 4 zur Aorta ascendens.

Insgesamt stellt das System ein biventrikuläres Assistenzsystem dar. Die beiden Pumpen 1, 4 sind als Rotationspumpen mit je einem Rotor ausgebildet, der mittels eines Elektromotors angetrieben wird und durch Rotation mit hoher Geschwindigkeit in Axialrichtung Blut fördert. Die Rotationsachsen der beiden Rotoren liegen parallel zu den in Figur 1 eingetragenen Pfeilen 9, 10, die jeweils die Flussrichtung des Blutes beim Betrieb der Pumpe andeuten. Es sind zwei Kabel 11, 12 eingezeichnet, die der Durchführung elektrischer Leitungen von den die Pumpen 1, 4 treibenden Elektromotoren zu einem gemeinsamen Steuergerät 13 dienen.

Die Pumpen 1, 4 können abgestimmt aufeinander betrieben werden, wobei periodische Schwankungen der Förderleistung zur Imitation einer pulsatilen organischen Herztätigkeit vorgesehen sein können. Innerhalb der verschiedenen Kammern des Herzens 8 oder in den Kanülen 2, 3, 5, 6 können Durchflusssensoren und/oder Drucksensoren vorgesehen sein, um die Förderleistung und den Druckabfall über den beiden Pumpen 1, 4 aufeinander abstimmen zu können.

In dem Steuergerät 13 werden die Sensordaten gesammelt und verarbeitet und gegebenenfalls unter Berücksichtigung von erfassten physiologischen Daten des Patienten in Steuerdaten für die Pumpen 1, 4 umgesetzt. Das Steuergerät 13 weist zu diesem Zweck wenigstens einen Mikrocontroller auf, der mit einer Speichereinheit versehen ist und mittels eines Computerprogramms betrieben wird. Die Programmierung des Steuergeräts 13 und/oder die Parametrisierung der Steuerung der Pumpen 1, 4 ist individuell möglich und auf den Patienten abstimmbar.

Figur 2 zeigt beispielhaft eine Rotationspumpe, wie sie beim Herzunterstützungssystem der Figur 1 vorgesehen ist. Die Pumpe 1 weist ein Gehäuse auf, in dem ein Rotor um die Rotationsachse rotiert, die in Figur 2 mit 14 bezeichnet ist. Eine elektrische Leitung ist durch das Kabel 11 bis zur Pumpe 1 geführt. Dort wird mittels der elektrischen Leitung über einen bestromten Stator ein Magnetfeld erzeugt, das einen innenliegenden Rotor bürstenlos antreibt. Der Rotor kann beispielsweise sowohl in der radialen als auch in der axialen Lagerung mittels Magnetlagern reibungsarm gelagert sein.

An die Pumpe 1 ist sowohl am Pumpeneinlass 15 als auch am Pumpenauslass 16 jeweils eine Kanüle mit relativ großem Querschnitt angeschlossen. Es kann sinnvoll oder notwendig sein, an diese groß dimensionierten Kanülen im Durchschnitt kleinere Kanülen anzuschließen, die zum Herzen des Patienten führen und durch die Haut des Patienten hindurchtreten. Da die Pumpen 1, 4 in zahlreichen Ausführungsbeispielen außerhalb des Patientenkörpers angeordnet werden, wird der Körper des Patienten durch groß dimensionierte Kanülen im Bereich der Pumpen nicht beeinträchtigt. Es sind jedoch Konnektoren notwendig, die den Übergang von einem großen Kanülendurchmesser zu einem kleinen Kanülendurchmesser ermöglichen.

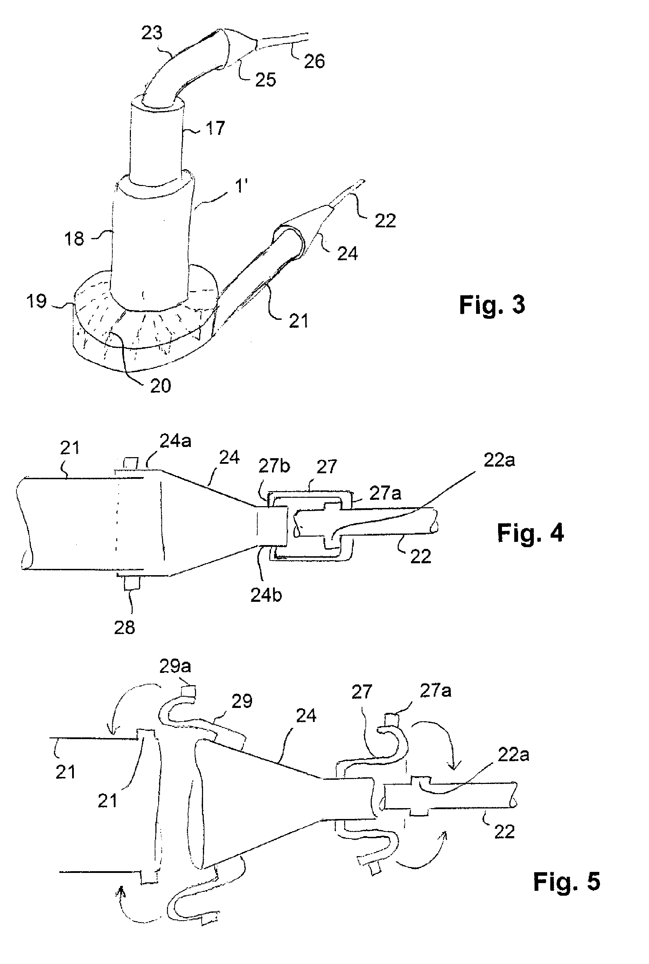

Figur 3 zeigt in einer dreidimensionalen Ansicht eine Pumpe 1', die als Radialpumpe ausgebildet ist. Dies bedeutet, dass über einen Pumpeneinlass 17 und einen axialen Einströmbereich 18 zu einem Rotor, der im Rotorgehäuse 19 angeordnet und gestrichelt dargestellt ist, die Flüssigkeit gefördert wird, wobei die Flüssigkeit durch den Rotor 20 zentrifugal in radialer Richtung gefördert und über einen Pumpenauslass 21 zu einer Kanüle 22 transportiert wird. Der Durchmesser des Pumpenauslasses 21 ist so groß, dass die Förderung der Flüssigkeit möglichst ungebremst geschehen kann. Die Pumpe 1' ist gemeinsam mit den Pumpenauslässen und den gezeigten Kanülen 21 am Pumpenauslass und 23 am Pumpeneinlass außerhalb des Patientenkörpers angeordnet.

Es sind Konnektoren 24, 25 vorgesehen, die den Fluidkanal, jeweils ausgehend von einem der Pumpe 1' näher liegenden Bereich zu einem der Pumpe 1' ferner liegenden Bereich, reduzieren. An die pumpenfernen Enden der Konnektoren 24, 25 sind jeweils im Durchmesser gegenüber den Kanülen 21, 23 reduzierte feinere Kanülen 22, 26 fluiddicht angeschlossen. Diese im Durchmesser (sowohl Innendurchmesser als auch Außendurchmesser) kleineren Kanülen 22, 26 führen durch die Haut des Patienten in den Patientenkörper und gegebenenfalls bis zum Patientenherzen oder den großen Blutgefäßen.

Auf der Oberfläche der Kanülen 22, 26 können Bereiche vorgesehen sein, die für den Durchtritt durch die Haut des Patienten besonders geeignet sind, beispielsweise durch eine Aufrauung an der Oberfläche oder durch Auflage einer Einwachsschicht, die das Anwachsen von organischem Gewebe besonders erleichtert.

Die Konnektoren 24, 25 sind in ihrer äußeren Erscheinung ebenso wie bezüglich ihres Innenraums zylindersymmetrisch konisch ausgestaltet, um in möglichst effizienter Art die Durchmesserreduktion des Fluidkanals zu gewährleisten.

Die Konnektoren 24, 25 sind mit den Kanülen 21, 23, 22, 26 mittels Verbindungselementen fluiddicht verbunden, die in Figur 3 nicht näher dargestellt sind.

Die Reduktion des Fluidkanals / der Fluidkanäle im Zuge der Durchmesserreduktion in den Konnektoren 24, 25 und dem weiteren Verlauf der feinen Kanülen 22, 26 bringt eine Erhöhung des Fließwiderstands der zu fördernden Flüssigkeit mit sich, den die Pumpe 1 bzw. 1' zusätzlich kompensieren muss.

Um den Fluidwiderstand möglichst zu reduzieren, kann die Länge der Kanülen 21, 23, die im Durchmesser nicht wesentlich kleiner gestaltet sind als die Pumpenein- und -auslässe, dahingehend optimiert werden, dass sie praktisch bis zum Körper des Patienten führen, worauf sich jeweils ein Konnektor 24, 25 und darauf folgend die feinen Kanülen 22, 26 anschließen, wobei der Hautdurchtritt durch die Haut des Patienten im pumpenseitigen Anfangsbereich der feinen Kanülen 22, 26 liegt, so dass nur wenige Zentimeter der Kanülen 22, 26, insbesondere weniger als 10 % der Gesamtlänge dieser Kanülen, aus dem Patientenkörper herausragen. Damit wird die Leistung der Pumpen, die zur Erzeugung des notwendigen Druckgefälles erforderlich ist, und damit bei der Verwendung von Rotationspumpen auch die Rotationsgeschwindigkeit möglichst weit reduziert. Dadurch können Blutschädigungen, die bei hohen Pumpendrehzahlen wahrscheinlicher werden können, vermieden werden.

Anhand der Figur 4 soll die Art der Verbindung der Konnektoren 24, 25 mit den Kanülen 21, 23, 22, 26 näher erläutert werden. In Figur 3 ist der Konnektor 24 mittig zwischen einer Kanüle 21 größeren Durchmessers und einer zweiten Kanüle 22 geringeren Durchmessers dargestellt. Die Kanüle 22, die am pumpenfernen Ende des Konnektors 24 angeordnet ist, weist sowohl bezüglich des Außendurchmessers als auch des Innendurchmessers einen geringeren Durchmesser auf als die Kanüle 21 am pumpennahen Ende des Konnektors 24.

Die Kanülen 21, 22 können aus einem biegsamen Kunststoff/Elastomer, beispielsweise einem Silikonelastomer, oder beispielsweise aus Polyäthylen hergestellt sein. Der Konnektor 24 besteht üblicherweise auch aus einem Kunststoff, der jedoch steifer sein kann als der Kunststoff, aus dem die Kanülen und/oder die Verbindungselemente bestehen.

Der Konnektor 24 weist an seinen beiden Enden jeweils zylindrische Ansatzstücke 24a, 24b auf, an denen die Verbindungselemente 27, 28 ansetzen. Dabei ist in dem gezeigten Beispiel das Verbindungselement 28 einfach als Spannring ausgestaltet, der über das Ansatzstück 24a geworfen und dort gespannt wird, so dass das Ansatzstück 24a auf den Außendurchmesser der Kanüle 21 fluiddicht aufgeklemmt wird. Die Verbindung kann auch derart gestaltet werden, dass die Kanüle außen auf das Ansatzstück 24a gezogen wird, was dann sinnvoll ist, wenn das Material der Kanüle 21 weicher ist als das Material des Konnektors 24. Über den Außendurchmesser der Kanüle 21 kann dann der Spannring 28 gespannt werden.

Im pumpenfernen Bereich des Konnektors 24 ist ein Verbindungselement 27 in Form eines Schlauchstücks mit zwei radial nach innen weisenden Krempen 27a, 27b gezeigt. Die Kanüle 22 kann bis in den Ansatz 24b hinein oder in eine Position außen auf dem Ansatz 24 aufsitzend geschoben werden. Das Verbindungselement 27 kann jedoch auch als eine Muffe ausgebildet sein, die die Verbindung zwischen dem Konnektor 24 und der Kanüle 22 herstellt, die für sich voneinander beabstandet sind. Jedoch muss dann darauf geachtet werden, dass in dem Verbindungselement 24 keine Totwassergebiete entstehen. Vorteilhaft wird deshalb eine unmittelbare Verbindung zwischen dem Konnektor und der Kanüle 22 sein.

Die Krempe 27b des Verbindungselements 27 kann elastisch auf das Ansatzstück 24 dichtend aufgeklemmt sein. Zur Verbindung mit einer Kanüle 22 kann das Verbindungselement 27 elastisch aufgeweitet werden, und zwar so weit, bis die Krempe 27a nach hinten über den zylindrischen Teil des Verbindungselements umgeschlagen wird. Eine derartige Stellung ist in der Figur 5 näher dargestellt. Wird das Verbindungselement 27 dann auf die Kanüle 22 aufgeschnappt, so kontrahiert die Krempe 27a elastisch und klemmt sich auf die zylindrische äußere Oberfläche der Kanüle 22 auf. Für einen besseren Zusammenhalt und eine zuverlässige Dichtung kann die Kanüle 22 einen umlaufenden Steg 22a aufweisen, hinter den sich die Krempe 27a legen kann.

In Figur 5 ist beispielhaft der Verbindungsvorgang gezeigt, bei dem zunächst der Konnektor 24 mit der Kanüle 21 und der Kanüle 22 zusammengesteckt wird, wobei die Verbindungselemente 27, 29 beide als umschlagbare Schnappringverbinder ausgebildet sind. Dies bedeutet, dass die dargestellte Position der Verbindungselemente 27, 29 für sich stabil ist, bis die freien Enden der Verbindungselemente 27, 29 mit den Krempen 27a, 29a bei der Herstellung der Verbindung beispielsweise von medizinischem Personal derart betätigt werden, dass die Verbinder hinter den Stegen 22a der Kanüle 22 und 21a der Kanüle 21 einschnappen, um so eine Abdichtung des Fluidkanals zu schaffen, der von der Kanüle 21 über den Konnektor 24 zur Kanüle 22 führt. Auf diese Weise ist beispielsweise während des Implantierungsvorgangs in einfacher Weise eine Verbindung herstellbar.

Das vorgestellte Herzunterstützungssystem erlaubt, auch wenn innerhalb des Patientenkörpers Kanülen mit einem geringen Innendurchmesser verwendet werden, wie beispielsweise bei Kleinkindern, außerhalb des Patientenkörpers die Verwendung von Rotorpumpen in Verbindung mit einem sich verjüngenden Querschnitt der verbindenden Fluidkanäle.

In den Herzkammern können Sensoren 30, 31 zur Messung des Drucks vorgesehen sein, die mit dem Steuergerät 13 verbunden sind, um die Pumpen 1, 4 abgestimmt anzusteuern.

## Patentansprüche

1. Herzunterstützungssystem mit zwei Pumpen (1, 4) und mit diesen verbundenen Kanülen, wobei eine erste Pumpe zur Verbindung mittels eines wenigstens teilweise durch zwei Kanülen (2, 3, 5, 6, 15, 16, 21, 22, 23, 26) gebildeten ersten Fluidkanals mit dem Rechtsherzsystem, insbesondere mit dem rechten Ventrikel und der Pulmonalarterie, eingerichtet ist, während die zweite Pumpe zur Verbindung mittels eines durch zwei weitere Kanülen gebildeten zweiten Fluidkanals mit dem Linksherzsystem, insbesondere mit dem linken Ventrikel einerseits und der Aorta andererseits, eingerichtet ist, **dadurch gekennzeichnet, dass** beide Pumpen (1, 4) als Rotationspumpen zur Anordnung außerhalb eines Patientenkörpers (7) ausgebildet sind.

2. Herzunterstützungssystem nach Anspruch 1, **dadurch gekennzeichnet, dass** im Verlauf von wenigstens einem Fluidkanal, der mit einer der Pumpen (1, 4) verbunden ist, ein Konnektor (24, 25) vorgesehen ist, in dem sich der Querschnitt des Fluidkanals mit wachsender Entfernung von der Pumpe verringert.

3. Herzunterstützungssystem nach Anspruch 3, **dadurch gekennzeichnet, dass** der Querschnitt des Fluidkanals innerhalb des Konnektors (24, 25) konisch ausgebildet ist.

4. Herzunterstützungssystem nach Anspruch 1, 2 oder 3, **dadurch gekennzeichnet, dass** der Konnektor (24, 25) aus einem steifen Material, insbesondere einem Kunststoff oder Metall, besteht.

5. Herzunterstützungssystem nach Anspruch 1, 2 oder 3, **dadurch gekennzeichnet, dass** der Konnektor (24, 25) aus einem elastomeren Material besteht.

6. Herzunterstützungssystem nach Anspruch 2 oder einem der folgenden, **dadurch gekennzeichnet, dass** der Konnektor (24, 25) mit einem Pumpenanschluss und/oder mit einer Kanüle (2, 3, 5, 6, 15, 16, 21, 22, 23, 26) mittels eines aus einem Elastomer bestehenden Verbindungselementes (27, 28, 29) fluiddicht verbunden ist.

7. Herzunterstützungssystem nach Anspruch 1 oder einem der folgenden, **dadurch gekennzeichnet, dass** wenigstens eine der Pumpen (1, 4) mit einem Graft verbunden ist.

8. Herzunterstützungssystem nach Anspruch 1 oder einem der folgenden, **dadurch gekennzeichnet, dass** die Kanülen jeweils einen Bereich aufweisen, der zum Durchtritt durch die Haut des Patienten eingerichtet ist.

9. Herzunterstützungssystem nach Anspruch 1 oder einem der folgenden, **dadurch gekennzeichnet, dass** die beiden Pumpen (1, 4) mit einem gemeinsamen Steuergerät (13) verbunden sind.

10. Herzunterstützungssystem nach Anspruch 9, **gekennzeichnet durch** wenigstens einen, insbesondere zwei Drucksensoren (30, 31), mit denen das Steuergerät (13) verbunden ist.

11. Herzunterstützungssystem nach Anspruch 10, **dadurch gekennzeichnet, dass** wenigstens einer der Drucksensoren (30, 31) in einem der Fluidkanäle oder in einem Herzventrikel, insbesondere je einer von wenigstens zwei Drucksensoren in einem der Fluidkanäle und in einem Herzventrikel angeordnet ist.

12. Herzunterstützungssystem nach Anspruch 2 oder einem der folgenden, **dadurch gekennzeichnet, dass** wenigstens ein Konnektor (24, 25) unmittelbar an einem Pumpeneinlass (17) oder -auslass angeordnet ist.

13. Computerprogrammprodukt, **gekennzeichnet durch** ein Programm zum Betrieb des Steuergerätes (13) derart, dass der Druck und/oder die Volumenströme in den beiden mit je einer der Pumpen (1, 4) verbundenen Fluidkanälen aufeinander abgestimmt sind.

14. Verfahren zum Betrieb eines Herzunterstützungssystems nach einem der Ansprüche 1 bis 11, **dadurch gekennzeichnet, dass** die Leistungen der beiden Pumpen (1, 4) derart gesteuert werden, dass der Druck und/oder die Volumenströme in den beiden mit je einer der Pumpen verbundenen Fluidkanälen aufeinander abgestimmt werden.

15. Herzunterstützungssystem mit mindestens einer Pumpe (1, 4) und mit dieser verbundenen Kanülen, wobei die mindestens eine Pumpe zur Verbindung mittels eines wenigstens teilweise durch wenigstens eine Kanüle (2, 3, 5, 6, 15, 16, 21, 22, 23, 26) gebildeten ersten Fluidkanals mit dem Rechtsherzsystem, insbesondere mit dem rechten Ventrikel und der Pulmonalarterie, eingerichtet ist oder zur Verbindung mittels eines wenigstens teilweise durch wenigstens eine Kanüle gebildeten zweiten Fluidkanals mit dem Linksherzsystem, insbesondere mit dem linken Ventrikel einerseits und der Aorta andererseits, eingerichtet ist, **dadurch gekennzeichnet, dass** die mindestens eine Pumpe (1, 4) als Rotationspumpe zur Anordnung außerhalb eines Patientenkörpers (7) ausgebildet ist.

16. Verfahren nach einem Anspruch 13, **dadurch gekennzeichnet, dass** die Leistungen der beiden Pumpen (1, 4) pulsatil gesteuert werden.
